(19)

**Europäisches Patentamt**
**European Patent Office**
**Office européen des brevets**

(11) **EP 2 208 497 A1**

(12) **EUROPEAN PATENT APPLICATION**

(43) Date of publication:
21.07.2010 Bulletin 2010/29

(51) Int Cl.:
*A61K 31/575* (2006.01)　　*A61P 35/00* (2006.01)
*A61P 43/00* (2006.01)

(21) Application number: 09150660.0

(22) Date of filing: 15.01.2009

(84) Designated Contracting States:
**AT BE BG CH CY CZ DE DK EE ES FI FR GB GR HR HU IE IS IT LI LT LU LV MC MK MT NL NO PL PT RO SE SI SK TR**
Designated Extension States:
**AL BA RS**

(71) Applicant: **Charité-Universitätsmedizin Berlin (Charité)**
**10117 Berlin (DE)**

(72) Inventor: **The designation of the inventor has not yet been filed**

(74) Representative: **Vossius & Partner**
**Siebertstrasse 4**
**81675 München (DE)**

(54) **Use of Ursodeoxycholic acid (UDCA) for enhancing the general health condition of a tumor patient**

(57)　The present invention relates to Ursodeoxycholic acid (UDCA) for use in ameliorating the general health condition of a tumor patient. 22% of cancer patients die of the cancer associated general health condition, which translates to approx. 1 million patients per year. The exact cause of death is unknown in these patients. In cancer model rats UDCA at 100mg/kg/d shows a strong improvement in body composition and body weight compared to placebo, which can also be seen in individual muscles and fat deposits. Cardiac function and survival is markedly improved by 100mg/kg/d and to a lesser degree by 25mg/kg/d.

EP 2 208 497 A1

**Description**

BACKGROUND OF THE INVENTION

Ursodiol, also known as ursodeoxycholic acid and the abbreviation UDCA, is one of the secondary bile acids, which are metabolic by-products of intestinal bacteria. Primary bile acids are produced by the liver and stored in the gall bladder. When secreted into the colon, primary bile acids can be metabolized into secondary bile acids by intestinal bacteria. Primary and secondary bile acids help the body digest fats. Ursodeoxycholic acid helps regulate cholesterol by reducing the rate at which the intestine absorbs cholesterol molecules while breaking up micelles containing cholesterol. Because of this property, ursodeoxycholic acid is used to treat (cholesterol) gallstones non-surgically.

While some bile acids are known to be colon tumor promoters (eg. deoxycholic acid), others such as ursodeoxycholic acid are chemopreventive, perhaps by inducing cellular differentiation and/or cellular senescence in colon epithelial cells.

Ursodeoxycholic acid has also been shown experimentally to suppress immune response such as immune cell phagocytosis. Increased quantities of systemic ursodeoxycholic acid can be toxic.

Bile acids are important signaling molecules that help regulate the regrowth of liver tissue. Ursodeoxycholic acid has been used as an experimental tool in liver regrowth studies. There is a possible link between the immune-repression and liver-regeneration properties of this substance, but clinical studies have yet to confirm these suspicions. Ursodeoxycholic acid goes by the trade names Actigall, Ursofalk, Urso, and Urso Forte. In Italy, it is marketed under the name Deursil.

Ursodeoxycholic acid can be chemically synthesized and was brought to market by the Montreal-based Axcan Pharma in 1998, which continues to market the drug.

It is used to treat primary biliary cirrhosis and other cholestatic diseases. In children it is used in biliary atresia, which is a cause of neonatal jaundice.

Ursodeoxycholic acid reduces elevated liver enzyme levels by facilitating bile flow through the liver and protecting liver cells.

Ursodeoxycholic acid is found in large quantities in bear bile.

UDCA is a bitter tasting white powder consisting of crystaline particles freely soluble in ethanol and glacial acetic acid, slightly soluble in chloroform, sparingly soluble in ether, and practically insoluble in water. UDCA is normally present in a minor fraction of the total bile acids of humans (about 5%). Following oral administration, the majority of UDCA is absorbed by passive diffusion but its absorption is incomplete. Once absorbed, the compound undergoes hepatic extraction to the extent of about 50% in the absence of liver disease. In the liver, it is conjugated with glycine or taurine, then secreted into the bile. The conjugates are absorbed in the small intestine by passive and active mechanisms. The conjugates can also be deconjugated in the ileum by intestinal enzymes.

22% of cancer patients die of the cancer associated general health condition, which translates to approx. 1 million patients per year. The exact cause of death is unknown in these patients.

To date there is no therapy for improving the general condition of tumor patients that effectively also prolongs life span.

SUMMARY OF THE INVENTION

The present inventors have astonishingly found that ursodeoxycholic acid (UDCA) may be used to ameliorate the general health condition of a tumor patient.

The present invention relates to ursodeoxycholic acid (UDCA) for use in ameliorating the general health condition of a tumor patient.

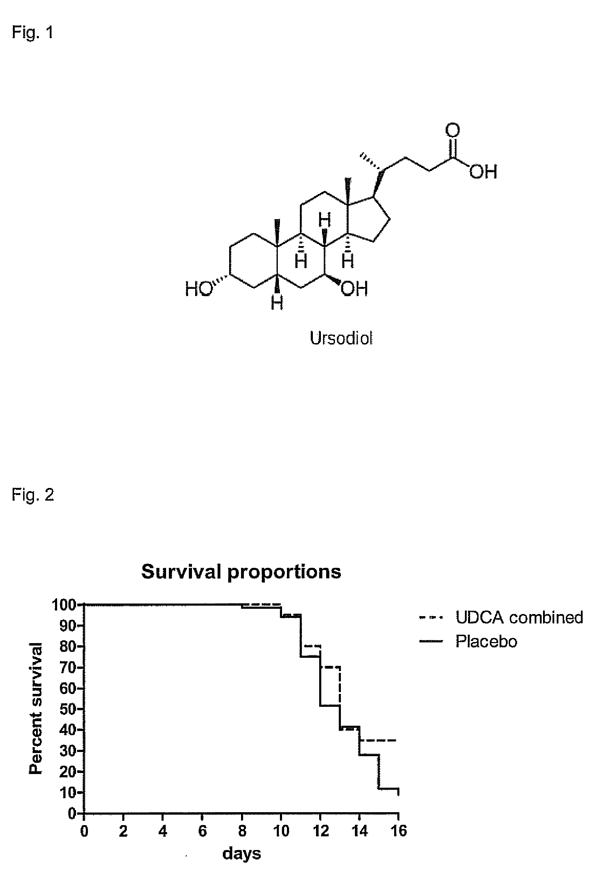

Herein, ursodiol or ursodeoxycholic acid (UDCA) is $3\alpha,7\beta$-dihydroxy-5$\beta$-cholan-24-oic acid; see also Fig. 1.

"Patients" in the meaning of the invention are understood to be all persons and animals, irrespective whether or not they exhibit pathological changes, unless stated otherwise. In a preferred embodiment the patient according to the invention is a human. In a further preferred embodiment of the invention the patient is a human suspected or proven to have a tumor.

Herein, ameliorating the general condition of a tumor patient means enhancing or bettering one or more of the following conditions, the cardiac condition, personal activity, food intake, body composition, body weight, and/or white fat amount. Some patients will react to UDCA by a bettering or enhancement of only one of the above. In other patients many of the above conditions will better. The direct effect is prolonged life span.

DETAILED DESCRIPTION OF THE INVENTION

The invention relates to Ursodeoxycholic acid (UDCA) for use in ameliorating the general health condition of a tumor patient.

The inventors have identified this effect in a tumor animal model. Ascites hepatoma Yoshida AH-130 cells ($10^8$) were given to male Wistar rats. The rats were then treated with UDCA. The inventors observed that one or more of the following conditions improved, the cardiac condition, personal activity, food intake, body composition, body weight, and/or white fat amount. Some rats reacted to UDCA by a bettering or enhancement of only one of the above. In other rats many of the above conditions improved. The direct effect is prolonged life span for the patient.

In the following we describe some of the effects of UDCA. There are various indicators for cardiac protective effects of a given substance, one being the left ventricular ejection fraction (LVEF) another the fractional shortening (FS), also the end-diastolic volume (EDV), as well as the stroke volume (SV) and the end-systolic diameter (ESD). For UDCA the inventors have astonishingly found in a rat tumor model that the administration of UDCA leads to substantially better cardiac values than in rats which were given a placebo. Also, body fat reduction dropped, food intake increased and activity rose in rats being administered with UDCA.

In cardiovascular physiology, ejection fraction (EF) is the fraction of blood pumped out of a ventricle with each heart beat. The term ejection fraction applies to both the right and left ventricles; one can speak equally of the left ventricular ejection fraction (LVEF) and the right ventricular ejection fraction (RVEF). Without a qualifier, the term ejection fraction refers specifically to that of the left ventricle.

By definition, the volume of blood within a ventricle immediately before a contraction is known as the end-diastolic volume. Similarly, the volume of blood left in a ventricle at the end of contraction is end-systolic volume. The difference between end-diastolic and end-systolic volumes is the stroke volume, the volume of blood ejected with each beat. Ejection fraction (Ef) is the fraction of the end-diastolic volume that is ejected with each beat; that is, it is stroke volume (SV) divided by end-diastolic volume (EDV):

$$E_f = \frac{SV}{EDV} = \frac{EDV - ESV}{EDV}$$

In a healthy 70-kg (154-lb) man, the SV is approximately 70 ml and the left ventricular EDV is 120 ml, giving an ejection fraction of 70/120, or 0.58 (58%). Right ventricular volumes being roughly equal to those of the left ventricle, the ejection fraction of the right ventricle is normally equal to that of the left ventricle within narrow limits.

Healthy individuals typically have ejection fractions greater than 0.55. However, normal values depend upon the modality being used to calculate the ejection fraction. Damage to the muscle of the heart (myocardium) such as that sustained during myocardial infarction or in cardiomyopathy impairs the heart's ability to eject blood and therefore reduces ejection fraction. This reduction in the ejection fraction can manifest itself clinically as heart failure. The ejection fraction is one of the most important predictors of prognosis; those with significantly reduced ejection fractions typically have a poorer prognosis.

Ejection fraction is commonly measured by echocardiography, in which the volumes of the heart's chambers are measured during the cardiac cycle. Ejection fraction can then be obtained by dividing stroke volume by end-diastolic volume as described above.

Other methods of measuring ejection fraction include cardiac MRI, fast scan cardiac computed axial tomography (CT) imaging, ventriculography, Gated SPECT, and the MUGA scan. A MUGA scan involves the injection of a radioisotope into the blood and detecting its flow through the left ventricle. The historical gold standard for the measurement of ejection fraction is echocardiopgraphy ventriculography. As can be seen in table 1 below UDCA, rats had much better left ventricular ejection fraction values LV EF values than placebo mice.

Ideally, the UDCA is in a pharmaceutical composition. This may be administered to a mammal by any route which provides a sufficient level of UDCA. It can be administered systemically or locally. Such administration may be parenterally, transmucosally, e.g., orally, nasally, rectally, intravaginally, sublingually, submucosally or transdermally. Preferably, administration is parenteral, e.g., via intravenous or intraperitoneal injection, and also including, but is not limited to, intraarterial, intramuscular, intradermal and subcutaneous administration. If the pharmaceutical composition of the present invention is administered locally it can be injected directly into the organ or tissue to be treated.

The pharmaceutical compositions of the invention may comprise a therapeutically effective amount of a compound and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeia for use in animals, and more particularly in humans. The term "carrier" refers to a diluent, adjuvant, excipient, or vehicle with which the therapeutic is administered. Such pharmaceutical carriers can be sterile liquids, such as saline solutions in water and oils, including those of petroleum, animal, vegetable or synthetic origin, such as peanut oil, soybean oil, mineral oil, sesame oil and the like. A saline solution is a preferred carrier when the pharmaceutical composition is administered intravenously. Saline solutions and aqueous dextrose and glycerol solutions can also be employed as liquid carriers, particularly for injectable solutions. Suitable pharmaceutical recipients include starch, glucose, lactose, sucrose, gelatin, malt, rice, flour, chalk, silica gel, so-dium stearate, glycerol monostearate, talc, sodium chloride, dried skim milk, glycerol, pro-pylene, glycol, water, ethanol and the like. The composition, if desired, can also contain minor amounts of wetting or emulsifying agents, or pH buffering agents. These compositions can take the form of solutions, suspensions, emulsion, tablets, pills, capsules, powders, sustained-release formulations and the like. The composition can be formulated as a suppository, with traditional binders and carriers such as triglycerides. The UDCA of the invention can be formulated as neutral or salt forms. Pharmaceutically acceptable salts include those formed with free amino groups such as those derived from hydrochloric, phosphoric, acetic, oxalic, tartaric acids, etc., and those formed with free carboxyl groups such as those derived from sodium, potassium, ammonium, calcium, ferric hydroxides, isopropylamine, triethyl-amine, 2-ethylamino ethanol, histidine, procaine, etc. Examples of suitable pharmaceutical carriers are described in "Remington's Pharmaceutical Sciences" by E. W. Martin. Such compositions will contain a therapeutically effective amount of the compound, preferably in purified form, together with a suitable amount of carrier so as to provide the form for proper administration to the patient. The formulation should suit the mode of administration.

Pharmaceutical compositions adapted for oral administration may be provided as capsules or tablets; as powders or granules; as solutions, syrups or suspensions (in aqueous or non-aqueous liquids); as edible foams or whips; or as emulsions. Tablets or hard gelatine capsules may comprise lactose, starch or derivatives thereof, magnesium stearate, sodium saccharine, cellulose, magnesium carbonate, stearic acid or salts thereof. Soft gelatine capsules may comprise vegetable oils, waxes, fats, semi-solid, or liquid polyols etc. Solutions and syrups may comprise water, polyols and sugars.

A problem when administering this active agent, a bile acid, to patients is its extreme bitterness. Preferably therefore, a taste-acceptable, liquid administration form for ursodeoxycholic acid with an adequately high active agent concentration is described. The liquid to be ingested is a suspension prepared accompanied by the addition of a swelling and/or thickening agent, which contains the active agent mainly in fine crystalline form as the disperse phase and only in a much smaller proportion dissolved in the aqueous dispersant (see also Canadian Patent 2130787).

An active agent intended for oral administration may be coated with or admixed with a material that delays disintegration and/or absorption of the active agent in the gastrointestinal tract (e.g., glyceryl monostearate or glyceryl distearate may be used). Thus, the sustained release of an active agent may be achieved over many hours and, if necessary, the active agent can be protected from being degraded within the stomach. Pharmaceutical compositions for oral administration may be formulated to facilitate release of an active agent at a particular gastrointestinal location due to specific pH or enzymatic conditions.

Pharmaceutical compositions adapted for transdermal administration may be provided as discrete patches intended to remain in intimate contact with the epidermis of the recipient for a prolonged period of time. Pharmaceutical compositions adapted for topical administration may be provided as ointments, creams, suspensions, lotions, powders, solutions, pastes, gels, sprays, aerosols or oils. For topical administration to the skin, mouth, eye or other external tissues a topical ointment or cream is preferably used. When formulated in an ointment, the active ingredient may be employed with either a paraffinic or a water-miscible ointment base. Alternatively, the active ingredient may be formulated in a cream with an oil-in-water base or a water-in-oil base. Pharmaceutical compositions adapted for topical administration to the eye include eye drops. In these compositions, the active ingredient can be dissolved or suspended in a suitable carrier, e.g., in an aqueous solvent. Pharmaceutical compositions adapted for topical administration in the mouth include lozenges, pastilles and mouthwashes.

Pharmaceutical compositions adapted for nasal administration may comprise solid carriers such as powders (preferably having a particle size in the range of 20 to 500 microns). Powders can be administered in the manner in which snuff is taken, i.e., by rapid inhalation through the nose from a container of powder held close to the nose. Alternatively, compositions adopted for nasal administration may comprise liquid carriers, e.g., nasal sprays or nasal drops. These compositions may comprise aqueous or oil solutions of the active ingredient. Compositions for administration by inhalation may be supplied in specially adapted devices including, but not limited to, pressurized aerosols, nebulizers or insufflators, which can be constructed so as to provide predetermined dosages of the active ingredient. In a preferred embodiment, pharmaceutical compositions of the invention are administered via the nasal cavity to the lungs.

Pharmaceutical compositions adapted for rectal administration may be provided as suppositories or enemas. Pharmaceutical compositions adapted for vaginal administration may be provided as pessaries, tampons, creams, gels, pastes, foams or spray formulations.

Pharmaceutical compositions adapted for parenteral administration include aqueous and non-aqueous sterile injectable solutions or suspensions, which may contain antioxidants, buffers, bacteriostats and solutes that render the compositions substantially isotonic with the blood of an intended recipient. Other components that may be present in such compositions include water, alcohols, polyols, glycerine and vegetable oils, for example. Compositions adapted for parenteral administration may be presented in unit-dose or multi-dose containers, for example sealed ampules and vials, and may be stored in a freeze-dried (lyophilized) condition requiring only the addition of a sterile liquid carrier, e.g., sterile saline solution for injections, immediately prior to use. Extemporaneous injection solutions and suspensions may be prepared from sterile powders, granules and tablets. In one embodiment, an autoinjector comprising an injectable solution of an UDCA may be provided for emergency use.

In a preferred embodiment, the composition is formulated in accordance with routine procedures as a pharmaceutical composition adapted for intravenous administration to human beings. Typically, compositions for intravenous administration are solutions in sterile isotonic aqueous buffer. Where necessary, the composition may also include a solubilizing agent and a local anesthetic such as lidocaine to ease pain at the site of the injection. Generally, the ingredients are supplied either separately or mixed together in unit dosage form, for example, as a dry lyophilized powder or water-free concentrate in a hermetically-sealed container such as an ampule or sachette indicating the quantity of active agent. Where the composition is to be administered by infusion, it can be dispensed with an infusion bottle containing sterile pharmaceutical grade water or saline. Where the composition is administered by injection, an ampule of sterile saline can be provided so that the ingredients may be mixed prior to administration. Oral formulations are preferred.

Suppositories generally contain active ingredient in the range of 0.5% to 10% by weight; oral formulations preferably contain 10% to 95% active ingredient.

A perfusate composition may be provided for use in transplanted organ baths, for in situ perfusion, or for administration to the vasculature of an organ donor prior to organ harvesting.

The invention also provides a pharmaceutical pack or kit comprising one or more containers filled with one or more of the ingredients of the pharmaceutical compositions of the invention. Optionally associated with such container(s) can be a notice in the form prescribed by a governmental agency regulating the manufacture, use or sale of pharmaceuticals or biological products, which notice reflects approval by the agency of manufacture, use or sale for human administration.

It is preferred that the ameliorating is characterized by an enhancement of the cardiac condition.

The ameliorating may also be characterized by a reduction of body weight loss and/or by an enhancement of activity.

The tumor according to the invention may be a cyst, an abscess, a cancer, a sarcoma, a carcinoma, a benign tumor or a malignant tumor.

Preferably the tumor is a cancer which is selected from the group comprising, melanoma cancer, lung cancer, prostate cancer, breast cancer, colon cancer, cervical cancer, brain cancer, kidney cancer, ovary cancer, pancreas cancer, cancer of the peritoneum, stomach cancer and skin cancer.

In one embodiment the ursodeoxycholic acid (UDCA) is tauro-ursodeoxycholic acid (t-UDCA) or glycoursodeoxycholic acid (GUDCA).

Ideally the UDCA is administered at a concentration of between 10 mg/kg/day and 200 mg/kg/day, more preferably at a concentration of between 15 mg/kg/day and 80 mg/kg/day, even more preferably at a concentration of between 15 mg/kg/day and 80 mg/kg/day, most preferably at a concentration of between 20 mg/kg/day and 40 mg/kg/day.

In a preferred embodiment the patient selected from treatment with UDCA displays weight loss of >5% over a period of 12 months or less, more preferably a weight loss of >7.5% over a period of 12 months or less and even more preferably of >10% over a period of 12 months or less. In cases were weight loss cannot be documented a BMI of less than 22 kg/m$^2$ is sufficient, or more preferably a BMI of less than 20 kg/m$^2$ is sufficient, or even more preferably a BMI of less than 18.5 kg/m$^2$ is sufficient.

In a preferred embodiment the patient selected from treatment with UDCA has a appendicle skeletal muscle index by DEXA of >5.45 kg/m$^2$ in females and <7.25 kg/m$^2$ in males. Alternatively the lean tissue depletion can be determined by mid upper arm circumference and the patients are selected from the group of patients that are <10th percentile for age and gender.

In a preferred embodiment the patient has a white fat concentration of less than 20%, or more preferably less than 16% or even more preferably less than 12%.

In a preferred embodiment the patient has a poor appetite, preferably a food intake <70% of normal food intake or even more preferably a limited food intake of only 20 kcal/kg body weight/d.

In a preferred embodiment the patient has a LV EF value of under 46%.

In an even more preferred embodiment the patient has a LV EF value of under 41 %. In an even more preferred embodiment the patient has a LV EF value of under 36%. In an even more preferred embodiment the patient has a LV EF value of under 31%. In an even more preferred embodiment the patient has a LV EF value of under 26%.

The invention also relates to a respective method of treatment as well as the use of UDCA for ameliorating the general health condition of a tumor patient.

Likewise, the invention also relates to the respective use of UDCA for the preparation of a medicament for ameliorating the general health condition of a tumor patient.

Preferably, ameliorating is characterized by an enhancement of the cardiac condition.

It is also preferred that ameliorating is characterized by a reduction of body weight loss.

It is further preferred that ameliorating is characterized by an enhancement of activity.

In a preferred embodiment of the use the tumor is a cyst, an abscess, a cancer, a sarcoma, a carcinoma, a benign tumor or a malignant tumor.

In a preferred embodiment of the use the tumor is a cancer selected from the group comprising melanoma cancer, lung cancer, prostate cancer, breast cancer, colon cancer, cervical cancer, brain cancer, kidney cancer, ovary cancer, pancreas cancer, cancer of the peritoneum, stomach cancer, urogenital cancer and skin cancer.

In a preferred embodiment of the ursodeoxcholic acid is tauro-usodeoxycholic acid (TUDCA) or glycoursodeoxycholic acid (GUDCA).

FIGURE CAPTIONS

Fig. 1 illustrates the structure of ursodiol, also known as ursodeoxycholic acid (UDCA).

Fig. 2 illustrates survival proportions of UDCA treated animals (dashed line) in comparison to placebo (solid line) as percent survival dependent on the day of treatment.

Fig. 3 illustrates survival proportions for two different UDCA dosages (25 mg/kg/d UDCA (grey line) and 100 mg/kg/d UDCA) (dashed line) in comparison to placebo (solid black line)

EXAMPLES

Study Plan

The ascites hepatoma Yoshida AH-130 cells ($10^8$) were inoculated into approx. 200g male Wistar rats. Alternatively animals received saline injection only (sham). The animals were housed in groups of three. The day after inoculation animals were randomized into various groups as described in the methods. The rats then received treatment with either placebo (n=78) or Ursodeoxycholic acid (25 and 100mg/kg/d, n=10, each) over a period maximal 16 days. The primary endpoints of the study included assessment of body weight, body composition and survival. Organ weight was assessed at the end of the study (or after death) as a secondary endpoint.

Methods

1. Animal model

Tumor inoculation ($10^8$ cell, AH-130 hepatoma) was performed in approx. 200g male Wistar rats. Alternatively, animals were injected with saline only. One day after tumor inoculation, the animals will start to receive treatment with UDCA or placebo per gavage.

Time line

Day -1:      Locomotor activity, food intake
Day 0:       NMR (Body composition), echocardiography
Day 1:       Tumor-inoculation
Day 2:       Randomisation, start therapy
Day 10/11:   Locomotor activity, food intake
Day 11:      echocardiography
Day 17:      end of therapy, sacrifice, storage of tissue and plasma, NMR (body composition, without tumor)

Results

1) 25mg/kg/d ursodeoxycholic acid

Animals treated with 25mg/kg/d ursodeoxycholic acid (UDCA) had an effect on body weight and body composition compared to placebo (weight loss reduction : 11.2%, lean loss reduction: 12.5%, fat loss reduction: 1 %). Individually muscle weight of *M. gastrocnemius* and *M. tibialis* were increased by 13.4% and 8.8%, respectively, compared to placebo. Epididymal fat mass was 178.6% higher in UDCA treated animals, while brown fat mass was increased by 16.3%. Heart weight was similar in UDCA- and placebo treated animals. Left ventricular (LV) ejection fraction was higher (22.9%), LV fractional shortening by 40.9% and LV stroke volume was unchanged compared to placebo (-3.4%), which implies less cardiac remodelling. Quality of life, assessed by food intake and spontaneous activity was immensely improved by 265.1% and unchanged (1.3%), respectively.

2) 100mg/kg/d ursodeoxycholic acid

Animals treated with 100mg/kg/d ursodeoxycholic acid (UDCA) showed a significant reduction of overall loss of body weight by 31.8% compared to placebo. Both, fat and lean mass, were partially protected by UDCA (lean loss reduction: 30.9%; fat loss reduction: 26.6% compared to placebo treated animals). Individually, muscle weight of the mixed fiber type muscles *M. gastronemius* and *M. tibialis* were increased by 11.5% and 11.6%, respectively, compared to placebo. Epididymal fat mass was 289.9% higher in UDCA treated animals, while brown fat mass was increased by 9.7%, which shows that the animals were able to preserve energy stores. Heart weight was 4.7% higher in UDCA-treated animals. Left ventricular (LV) ejection fraction was higher (17.9%), LV fractional shortening by 34.1 % and LV stroke volume by 45.7% leading to an overall better cardiac function. Quality of life, assessed by food intake and spontaneous activity was improved by 51.2% and by 4.4%, respectively, which points to a general improved well-being of the treated animals. Ultimately all improvements mentioned above have a strong and positive effect on outcome. The combined survival analysis of both treated groups show a 43% lower risk of death compared to placebo.

Table 1

|  | Placebo | UDCA 25mg | % vs Plac | UDCA 100mg | % vs Plac |
|---|---|---|---|---|---|
| n | 78 | 10 |  | 10 |  |
| Δ Body weight [g] | -53.7 ± 1.8 | -47.7 ± 8.9 | -11.2 | -36.6 ± 13.8* | -31.8 |
| Δ Lean mass [g] | -39.8 ± 1.6 | -34.8 ± 6.7 | -12.5 | -27.5 ± 9.7* | -30.9 |
| Gastrocnemius [mg] | 729.8 ± 12.1 | 827.8 ± 72.7* | 13.4 | 813.5 ± 80.5 | 11.5 |
| Tibialis [mg] | 269.1 ± 4.6 | 292.9 ± 20.4 | 8.8 | 300.3 ± 28.7 | 11.6 |
| Δ Fat mass [g] | -12.4 ± 0.4 | -12.3 ± 1.9 | -1 | -9.1 ± 3.2* | -26.6 |
| White fat [mg] | 112.3 ± 19.3 | 312.9 ± 39.3*** | 178.6 | 437.9±240.5** | 289.9 |
| Brown fat [mg] | 87.6 ± 3.0 | 101.9 ± 7.7 | 16.3 | 96.1 ± 14.0 | 9.7 |
| Food intake, day 11 [g/d] | 4.3 ± 0.5 | 15.7 ± 9.7** | 265.1 | 6.5 ± 3.1 | 51.2 |
| Activity, day 11 [counts/d) | 29509 ± 1775 | 30038 ± 6384 | 1.8 | 30796 ± 7734 | 4.4 |
| Heart [mg] | 505.7 ± 8.0 | 512.1 ± 19.2 | 1.3 | 529.4 ± 32.9 | 4.7 |
| LV EF, day 11 [%] | 51.9 ± 2.0 | 63.8 ± 5.6* | 22.9 | 61.2 ± 5.4 | 17.9 |
| LV FS, day 11 [%] | 30.8 ± 1.6 | 43.4 ± 4.1** | 40.9 | 41.3 ± 4.8* | 34.1 |
| LV EDV, day 11 [μL] | 196.6 ± 9.8 | 155.5 ± 37.8 | -20.9 | 238.7 ± 46.3 | 21.4 |
| LV SV, day 11 [μL] | 104.9 ± 6.9 | 101.3 ± 36,7 | -3.4 | 162.7 ± 41.4 | 45.7 |
| LV ESD, day 11 [mm] | 3.94 ± 0.11 | 2.91 ± 0.38** | -26.1 | 3.56 ± 0.19 | -9.7 |
| LV: left ventricular, EF: ejection fraction, FS: fractional shortening, EDV: end-diastolic volume, SV: stroke volume, ESD: end-systolic diameter. *: $p<0.05$, **:$p<0.01$, ***:$p<0.001$. |  |  |  |  |  |

3) Survival:

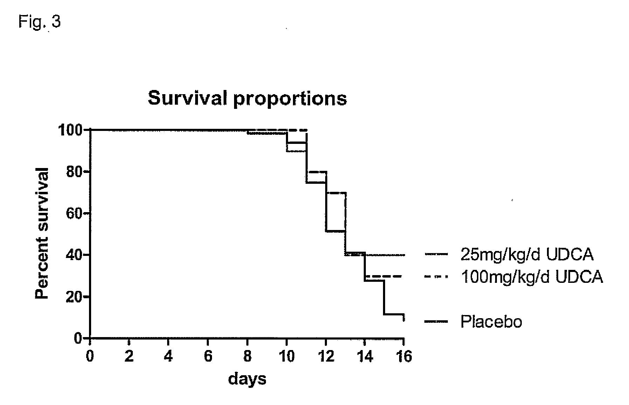

Survival proportions are shown for UDCA-treated animals in comparison to placebo-treated animals and for different dosages in Fig. 2 and 3, respectively.

UDCA vs Placebo: HR: 0.57 95% CI: 0.31-1.04 p=0.0685

This shows a 43% reduction in mortality.

Survival Proportions at day 16:

| | | |
|---|---|---|
| UDCA (n= 20): 35% | 95%CI: | 14-56% |
| Placebo (n= 68): 9% | 95%CI: 2-16% | |

| | | | |
|---|---|---|---|
| 25 mg/kg/d UDCA vs PlaGebo: | HR:0.53 | 95% CI: 0.24-1.15 | p=0.1069 |
| 100 mg/kg/d UDCA vs Placebo: | HR: 0.64 | 95% CI:0.29-1.40 | p=0.26 |

Survival Proportions:

| | | |
|---|---|---|
| 25 mg/kg/d UDCA (n= 10): | 40% | 95%CI: 10-70% |
| 100 mg/kg/d UDCA (n= 10): | 30% | 95%CI: 2-58% |
| Placebo (n= 68): | 9% | 95%CI: 2-16% |

The instantaneous hazard rate (HR) is the limit of the number of events per unit time divided by the number at risk as the time interval decreases.

$$h(t) = \lim_{\Delta t \to 0} \frac{\text{observed events}(t)/N(t)}{\Delta t}$$

where N(t) is the number at risk at the beginning of an interval.

The hazard ratio is the effect on this hazard rate of a difference, such as group membership (for example, treatment or control, male or female), as estimated by regression models which treat the log of the hazard rate as a function of a baseline hazard h0(t) and a linear combination of explanatory variables:

$$\log h(t) = f(h_0(t), \alpha + \beta_1 X_1 + \cdots + \beta_k X_k).$$

22% of cancer patients die of the cancer associated cachexia, which translates to approx. 1 million patients per year. The exact cause of death is unknown in these patients. Hence, patients are not receiving specialized therapy, as there is no causal treatment of even an established treatment regime for the symptoms by both physician and cancer societies. In cancer model rats UDCA at 100mg/kg/d shows a strong improvement in body composition and body weight compared to placebo, which can also be seen in individual muscles and fat deposits. Cardiac function is markedly improved by 100mg/kg/d and to a lesser degree by 25mg/kg/d. We have previously shown that cancer cachexia strongly affects heart function. We also show a 43% reduction in mortality in the combined groups compared to placebo.

**Claims**

1. Ursodeoxycholic acid (UDCA) for use in ameliorating the general health condition of a tumor patient.

2. Ursodeoxycholic acid according to claim 1, wherein the ameliorating is **characterized by** an enhancement of the cardiac condition.

3. Ursodeoxycholic acid according to claim 1, wherein the ameliorating is **characterized by** a reduction of body weight loss.

4. Ursodeoxycholic acid according to claim 1, wherein the ameliorating is **characterized by** an enhancement of activity.

5. Ursodeoxycholic acid according to any of the preceding claims, wherein the tumor is a cyst, an abscess, a cancer, a sarcoma, a carcinoma, a benign tumor or a malignant tumor.

6. Ursodeoxycholic acid according to claim 5, wherein the tumor is a cancer selected from the group comprising, melanoma cancer, lung cancer, prostate cancer, breast cancer, colon cancer, cervical cancer, brain cancer, kidney cancer, ovary cancer, pancreas cancer, cancer of the peritoneum , prostate cancer, stomach cancer urogenital cancer and skin cancer.

7. Ursodeoxycholic acid according to any of the claims above, wherein the ursodeoxycholic acid (UDCA) is tauro-ursodeoxycholic acid (TUDCA) or glycoursodeoxycholic acid (GUDCA).

8. Ursodeoxycholic acid according to any of the claims above, wherein the UDCA is administered at a concentration of between 10 mg/kg/day and 200 mg/kg/day.

9. Ursodeoxycholic acid according to any of the claims above, wherein the UDCA is administered at a concentration of between 15 mg/kg/day and 80 mg/kg/day.

10. Ursodeoxycholic acid according to any of the claims above, wherein the UDCA is administered at a concentration of between 20 mg/kg/day and 40 mg/kg/day.

11. Use of Ursodeoxycholic acid for the preparation of a medicament for ameliorating the general health condition of a tumor patient.

Fig. 1

Ursodiol

Fig. 2

Fig. 3

**Survival proportions**

Europäisches
Patentamt

European
Patent Office

Office européen
des brevets

**EUROPEAN SEARCH REPORT**

Application Number

EP 09 15 0660

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | INOUE W ET AL: "Effect of ursodeoxycholic acid on hepatotoxicity induced by flutamide during the treatment of prostatic cancer" NISHINIHON JOURNAL OF UROLOGY, vol. 59, no. 5, May 1997 (1997-05), pages 447-449, XP009118338 ISSN: 0029-0726 * abstract * | 1,3-6, 8-11 | INV. A61K31/575 A61P35/00 A61P43/00 |
| Y | | 1-11 | |
| X | US 2007/072828 A1 (YOO SEO H [US]) 29 March 2007 (2007-03-29) * abstract; claims 8,29; examples 9,10 * | 1,3-6, 8-11 | |
| Y | | 1-11 | |
| X | KOJIMA MUNEKADO ET AL: "Clinical utility of ursodeoxycholic acid in preventing flutamide-induced hepatopathy in patients with prostate cancer : A preliminary study" INTERNATIONAL JOURNAL OF UROLOGY, vol. 9, no. 1, 1 January 2002 (2002-01-01), pages 42-46, XP009118314 ISSN: 0919-8172 * abstract * | 1,4-6, 8-11 | TECHNICAL FIELDS SEARCHED (IPC) A61K |
| X | ICHIMURA, EIJI ET AL: "Improvement of flutamide-induced hepatotoxicity by ursodeoxycholic acid in male rats" KANZO, vol. 40, no. 4, 1999, pages 227-234, XP009118341 ISSN: 0451-4203 * abstract * | 1,3,5,6, 8-11 | |

-/--

The present search report has been drawn up for all claims

| Place of search | Date of completion of the search | Examiner |
|---|---|---|
| Munich | 17 June 2009 | Madalinska, K |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

Europäisches
Patentamt

European
Patent Office

Office européen
des brevets

# EUROPEAN SEARCH REPORT

Application Number

EP 09 15 0660

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 5 814 625 A (LARSON MARK V [US] ET AL) 29 September 1998 (1998-09-29) * abstract * * examples 1,2 * | 1,4-11 | |
| X | NARISAWA T ET AL: "Inhibitory effects of ursodeoxycholic acid on N-methylnitrosourea-induced colon carcinogenesis and colonic mucosal telomerase activity in F344 rats" JOURNAL OF EXPERIMENTAL AND CLINICAL CANCER RESEARCH, vol. 18, no. 2, 1 January 1999 (1999-01-01), pages 259-266, XP009118316 ISSN: 0392-9078 * abstract * | 1,4-6, 8-11 | |
| X | WO 97/18816 A (CHILDRENS HOSP MEDICAL CENTER [US]) 29 May 1997 (1997-05-29) * abstract; claims 1-7,25-31 * | 1,5-11 | |
| X | JP 2003 012519 A (MITSUBISHI PHARMA CORP) 15 January 2003 (2003-01-15) * abstract * | 1,4-6, 8-11 | TECHNICAL FIELDS SEARCHED (IPC) |
| X | JP 05 279259 A ((TANB  ) TOKYO TANABE CO) 26 October 1993 (1993-10-26) * abstract * | 1,4-6, 8-11 | |
| X | WO 00/48577 A (FALK PHARMA GMBH [DE]; OTTERBECK NORBERT [DE]) 24 August 2000 (2000-08-24) * abstract; claims 1-14 * | 1,4-6, 8-11 | |

-/--

The present search report has been drawn up for all claims

| Place of search | Date of completion of the search | Examiner |
|---|---|---|
| Munich | 17 June 2009 | Madalinska, K |

EPO FORM 1503 03.82 (P04C01)

## EUROPEAN SEARCH REPORT

Application Number

EP 09 15 0660

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | WO 00/53224 A (MAX DELBRUECK CENTRUM [DE]; ANKER STEFAN [DE]; COATS ANDREW [GB]; VOLK) 14 September 2000 (2000-09-14) * abstract; claims 1-75; examples 11,12 * ----- | 1-11 | |
| A | SKARLOS D V ET AL: "Megestrol acetate in cancer patients with anorexia and weight loss. A Hellenic Co-operative Oncology Group (HeCOG) study" ACTA ONCOLOGICA, vol. 32, no. 1, 1 January 1993 (1993-01-01), pages 37-41, XP008102133 ISSN: 0284-186X * abstract * ----- | 1-11 | |

TECHNICAL FIELDS
SEARCHED    (IPC)

The present search report has been drawn up for all claims

| Place of search | Date of completion of the search | Examiner |
|---|---|---|
| Munich | 17 June 2009 | Madalinska, K |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

&  : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 09 15 0660

This annex lists the patent family members relating to the patent documents cited in the above-mentioned European search report.
The members are as contained in the European Patent Office EDP file on
The European Patent Office is in no way liable for these particulars which are merely given for the purpose of information.

17-06-2009

| Patent document cited in search report | | Publication date | Patent family member(s) | | Publication date |
|---|---|---|---|---|---|
| US 2007072828 | A1 | 29-03-2007 | NONE | | |
| US 5814625 | A | 29-09-1998 | CA | 2193579 A1 | 23-09-1997 |
| | | | US | 5843929 A | 01-12-1998 |
| WO 9718816 | A | 29-05-1997 | AT | 397450 T | 15-06-2008 |
| | | | AU | 709594 B2 | 02-09-1999 |
| | | | AU | 7737796 A | 11-06-1997 |
| | | | BR | 9611606 A | 24-10-2000 |
| | | | CA | 2238040 A1 | 29-05-1997 |
| | | | CN | 1211185 A | 17-03-1999 |
| | | | EP | 0871452 A2 | 21-10-1998 |
| | | | JP | 3836148 B2 | 18-10-2006 |
| | | | JP | 2000500764 T | 25-01-2000 |
| | | | JP | 2006117698 A | 11-05-2006 |
| | | | NO | 982281 A | 08-07-1998 |
| | | | NZ | 323274 A | 28-07-2000 |
| | | | PL | 326931 A1 | 09-11-1998 |
| | | | US | 6251884 B1 | 26-06-2001 |
| | | | US | 5763435 A | 09-06-1998 |
| JP 2003012519 | A | 15-01-2003 | NONE | | |
| JP 5279259 | A | 26-10-1993 | JP | 3006695 B2 | 07-02-2000 |
| WO 0048577 | A | 24-08-2000 | DE | 19906290 A1 | 17-08-2000 |
| WO 0053224 | A | 14-09-2000 | AT | 365043 T | 15-07-2007 |
| | | | AU | 3809900 A | 28-09-2000 |
| | | | AU | 4105600 A | 28-09-2000 |
| | | | DE | 60035262 T2 | 21-02-2008 |
| | | | DK | 1158989 T3 | 22-10-2007 |
| | | | WO | 0053165 A2 | 14-09-2000 |
| | | | EP | 1158989 A2 | 05-12-2001 |
| | | | EP | 1212064 A2 | 12-06-2002 |
| | | | PT | 1158989 E | 01-10-2007 |
| | | | US | 2007197485 A1 | 23-08-2007 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CA 2130787